# EUROPEAN PATENT APPLICATION

(11) **EP 4 744 723 A1**
(43) Date of publication of application: **20.05.2026**
(21) Application number: 25214234.4
(22) Date of filing: 07.11.2025
(51) Int. Cl.: A61N 5/10

(54) **SYSTEMS AND METHODS FOR GENERATING AND EVALUATING RADIATION THERAPY TREATMENT PLANS**

(30) Priority: 15.11.2024 US 202418949305
(71) Applicant: Siemens Healthineers International AG, 6312 Steinhausen (CH)
(72) Inventor: KUUSELA, Esa, 02320 Espoo (FI); LANSONNEUR, Pierre, 69004 Lyon (FR); FOLKERTS, Michael, Costa Mesa, 92626 (US); MAGLIARI, Anthony, Newark, 60541 (US); KRIEGER, Miriam, 5235 Rüfenach (CH)
(74) Representative: Mathisen & Macara LLP

(57) **Abstract**

Provided herein are system (100) for evaluating candidate radiation therapy treatment plans. In an example, a system (100) can include at least one processor configured to obtain (202) data associated with a three-dimensional (3D) scan of a portion of a patient and at least one optimization objective; generate (204) a set of radiation therapy treatment plans based on the 3D scan; and evaluate (206) each radiation therapy treatment plan of the set of radiation therapy treatment plans based on a kernel function. The kernel function can include a metric evaluation function and a modification function that is based on at least one optimization objective. In examples, the at least one processor can be configured to determine (208) a selected radiation therapy treatment plan from among the set of radiation therapy treatment plans based on comparing a cost of the selected radiation therapy treatment plan to the costs of each of the radiation therapy treatment plans.

## Description

### TECHNICAL FIELD

This application relates generally to systems and methods for evaluating candidate radiation therapy (RT) treatment plans to be implemented when treating a patient and, in some non-limiting embodiments, to systems and methods for optimizing the evaluation of candidate RT treatment plans in accordance with dynamic heuristic parameters that are dependent on one or more optimization objectives.

### BACKGROUND

Radiation therapy (also referred to as "radiotherapy" or "RT") involves the delivery of radiation (energy) to targets within the body of a patient. For example, a multi-leaf collimator (MLC) coupled to a linear accelerator (LINAC) can be configured to move relative to a patient in accordance with a treatment plan to deliver energy to target tissue (e.g., tumors) at multiple control points. At each control point, the leaves of the MLC can be positioned and repositioned to form the shape of the beam generated by the LINAC. The goal of carefully planning the operation of the LINAC and shaping the beams using the MLC is to deliver radiation to the target tissue while minimizing the delivery of radiation to surrounding healthy tissue.

But conventional methods for evaluating treatment plans for controlling operation of the LINAC typically include use of an automated treatment planner to evaluate individual treatment plans within a given search space and determine a cost for each. For example, an automated treatment planner can calculate a cost based on a cost function gradient that, by extension, requires a gradient calculation for each individual term of the cost function. Where the cost function is represented as a sum of piecewise linear functions corresponding to various aspects of an RT plan being evaluated, the automated treatment planner can evaluate a set of treatment plans that are known to be globally acceptable, as evidenced when the cost remains below a cost threshold, but locally unacceptable as evidenced by one or more individual terms resulting in costs that are above corresponding local cost thresholds. This can result in unnecessary evaluation of treatment plans that, by extension, results in wasted computing resources and delayed convergence on an optimal treatment plan.

### SUMMARY

In accordance with a first aspect of the invention, there is provided a system as defined by claim 1.

In accordance with a second aspect of the invention, there is provided a method as defined by claim 7.

In accordance with a third aspect of the invention, there is provide a non-transitory computer-readable medium as defined by claim 12.

Optional features are defined by the dependent claims.

For the aforementioned reasons, there is a need for systems and methods that improve upon the treatment plan evaluation process and, more specifically, allow treatment planners to converge on optimal treatment plans quicker and with fewer evaluations.

Implementation of the systems and methods discussed herein can improve the performance of a computer executing a computer model (such as a treatment planner) tasked with generating a radiotherapy treatment plan. For instance, the treatment planner can be configured to converge on a result faster than would otherwise be possible using conventional computer models and/or computer models using conventional methods/systems. And by virtue of implementing the systems and methods discussed herein, with the dynamic acceleration of convergence of treatment plans as described herein, the same or better results can be achieved by the described systems while consuming less computing power than would otherwise be required by conventional systems.

In an embodiment, a system can include one or more processors configured to: obtain data associated with a three-dimensional (3D) scan of a portion of a patient and at least one optimization objective; generate a set of radiation therapy (RT) treatment plans based on the 3D scan of the portion of the patient; and evaluate each RT treatment plan of the set of RT treatment plans based on a kernel function to generate RT treatment plan costs for each corresponding RT treatment plan. Evaluation of each RT treatment plan can involve updating an output of the kernel function for each RT treatment plan based on a modification function that is based on the at least one optimization objective. In some implementations, the one or more processors can be configured to determine a selected RT treatment plan from among the set of RT treatment plans based on comparing the RT treatment plan costs of the selected RT treatment plan to the RT treatment plan costs of each of the RT treatment plans. In implementations, the one or more processors can be configured to provide data associated with the selected RT treatment plan to control operation of a linear accelerator (LINAC) during one or more phases of treatment of the patient.

In examples, the one or more processors configured to evaluate each RT treatment plan of the set of RT treatment plans can be configured to select the kernel function based on one or more aspects associated with a planning target volume represented by the 3D scan; and evaluate each RT treatment plan based on the kernel function, where the modification function is configured to update metric values output by the kernel function that are outside of a threshold range of metric values. In some examples, the one or more processors configured to update the metric values output by the kernel function outside of the threshold range of metric values can be configured to: update the metric values outside of the threshold range by a first factor such that consecutive metric values outside of the threshold range are updated at an exponential or logarithmic rate.

In some examples, the one or more processors configured to evaluate each RT treatment plan of the set of RT treatment plans can be configured to: evaluate each RT treatment plan based on the kernel function, where the modification function is configured to update a first set of values output by the kernel function that are outside of the threshold range of metric values by a first factor, and a second set of values output by the kernel function that are outside of the threshold range of metric values by a second factor. In examples, the one or more processors configured to evaluate each RT treatment plan of the set of RT treatment plans can be configured to evaluate each RT treatment plan based on the kernel function, where the modification function is configured to update a third set of values that satisfy the threshold range of metric value by a third factor that is less than the first factor and the second factor.

In some examples, the one or more processors configured to evaluate each RT treatment plan of the set of RT treatment plans can be configured to evaluate each RT treatment plan based on the kernel function, where the modification function is configured to update metric values output by the kernel function based on a functional form of the cost function. The functional form of the cost function can represent rates of change corresponding to dose values of energy delivered to a planning target volume or an organ at risk.

In some examples, the techniques described herein relate to a method including: obtaining, by at least one processor, data associated with a three-dimensional (3D) scan of a portion of a patient and at least one optimization objective; generating, by the at least one processor, a set of radiation therapy (RT) treatment plans based on the 3D scan of the portion of the patient; and evaluating, by the at least one processor, each RT treatment plan of the set of RT treatment plans based on a kernel function to generate RT treatment plan costs for each corresponding RT treatment plan. In some implementations, evaluation of each RT treatment plan can involve updating an output of the kernel function for each RT treatment plan based on a modification function that is based on the at least one optimization objective. In some implementations, the method can include determining, by the at least one processor, a selected RT treatment plan from among the set of RT treatment plans based on comparing the RT treatment plan costs of the selected RT treatment plan to the RT treatment plan costs of each of the RT treatment plans; and optionally providing, by the at least one processor, data associated with the selected RT treatment plan to control operation of a linear accelerator (LINAC) during one or more phases of treatment of the patient.

In some examples, the evaluating each RT treatment plan of the set of RT treatment plans can include selecting, by the at least one processor, the kernel function based on one or more aspects associated with a planning target volume represented by the 3D scan, and evaluating, by the at least one processor, each RT treatment plan based on the kernel function. The modification function can be configured to update metric values output by the kernel function that are outside of a threshold range of metric values. In examples, updating the metric values output by the kernel function outside of the threshold range of metric values can include updating, by the at least one processor, the metric values outside of the threshold range by a first factor such that consecutive metric values outside of the threshold range are updated at an exponential or logarithmic rate.

In some examples, the techniques described herein relate to a method, wherein evaluating each RT treatment plan of the set of RT treatment plans includes: evaluating, by the at least one processor, each RT treatment plan based on the kernel function, where the modification function is configured to update a first set of values output by the kernel function that are outside of the threshold range of metric values by a first factor, and a second set of values output by the kernel function that are outside of the threshold range of metric values by a second factor. In examples, evaluating each RT treatment plan of the set of RT treatment plans can include evaluating, by the at least one processor, each RT treatment plan based on the kernel function, where the modification function is configured to update a third set of values that satisfy the threshold range of metric value by a third factor that is less than the first factor and the second factor.

In some examples, evaluating each RT treatment plan of the set of RT treatment plans can include evaluating, by the at least one processor, each RT treatment plan based on the kernel function, where the modification function is configured to update metric values output by the kernel function based on a functional form associated with the modification function. In examples, the functional form associated with the modification function represents rates of change corresponding to dose values of energy delivered to a planning target volume or an organ at risk.

In some embodiments, a non-transitory computer-readable medium storing instructions thereon is described. The instructions, when executed by at least one processor, can cause the at least one processor to obtain data associated with a three-dimensional (3D) scan of a portion of a patient and at least one optimization objective; generate a set of radiation therapy (RT) treatment plans based on the 3D scan of the portion of the patient; and evaluate each RT treatment plan of the set of RT treatment plans based on a kernel function to generate RT treatment plan costs for each corresponding RT treatment plan. In some implementations, evaluation of each RT treatment plan can involve updating an output of the kernel function for each RT treatment plan based on a modification function that is based on the at least one optimization objective. In some implementations, the instructions can cause the at least one processor to determine a selected RT treatment plan from among the set of RT treatment plans based on comparing the RT treatment plan costs of the selected RT treatment plan to the RT treatment plan costs of each of the RT treatment plans, and provide data associated with the selected RT treatment plan to control operation of a linear accelerator (LINAC) during one or more phases of treatment of the patient.

In some examples, the instructions that cause the one or more processors to evaluate each RT treatment plan of the set of RT treatment plans can cause the one or more processors to select the kernel function based on one or more examples associated with a planning target volume represented by the 3D scan, and evaluate each RT treatment plan based on the kernel function. In some implementations, the modification function can be configured to update metric values output by the kernel function that are outside of a threshold range of metric values.

In examples, the instructions that cause the one or more processors to update the metric values output by the kernel function outside of the threshold range of metric values can cause the one or more processors to update the metric values outside of the threshold range by a first factor such that consecutive metric values outside of the threshold range are updated at an exponential or logarithmic rate. In some examples, the instructions that cause the one or more processors to evaluate each RT treatment plan of the set of RT treatment plans can cause the one or more processors to evaluate each RT treatment plan based on the kernel function. The modification function can be configured to update a first set of values output by the kernel function that are outside of the threshold range of metric values by a first factor, and a second set of values output by the kernel function that are outside of the threshold range of metric values by a second factor.

In examples, the instructions that cause the one or more processors to evaluate each RT treatment plan of the set of RT treatment plans can cause the one or more processors to evaluate each RT treatment plan based on the kernel function, and the modification function can be configured to update a third set of values that satisfy the threshold range of metric value by a third factor that is less than the first factor and the second factor. In some examples, the instructions that cause the one or more processors to evaluate each RT treatment plan of the set of RT treatment plans can cause the one or more processors to evaluate each RT treatment plan based on the kernel function, where the modification function is configured to update metric values output by the kernel function based on a functional form associated with the modification function.

By virtue of the implementation of the techniques described in associated with the systems and methods described herein, treatment plans can be evaluated in accordance with dynamic heuristic parameters that are dependent on one or more optimization objectives. This, in turn, can enable the treatment planners to converge on an optimal treatment plan faster by expediting the evaluation of candidate treatment plans within a search space. For example, where a given metric (e.g., dose delivered to an organ at risk) indicates that a predetermined threshold is satisfied for a given treatment plan (e.g., that the dose delivered to the organ at risk has reached a maximum desired dose), the cost function can be changed to use more accurately evaluated metrics. This can result in treatment plans having successively higher values for the given metric satisfying a cost threshold faster than would otherwise be possible, constraining the search space without setting fixed thresholds and preventing the treatment planner from evaluating borderline treatment plans.

The step of determining a selected RT treatment plan from among the set of RT treatment plans may be based on comparing the cost of each RT treatment plan with the cost of each other RT treatment plan, and selecting a RT treatment plan on the basis of the comparison.

The modification function may modify the treatment plan costs in accordance with a dynamic value that depends on metric values output by the kernel function.

The modification function may be dynamic and/or heuristic.

The modification function may update a metric value output by a kernel function in dependence on where the metric value is with respect to a threshold range of the metric value.

The modification function may be exponential or logarithmic.

A functional form of the modification function may comprise the modification function updating a metric value output by a kernel function as a function of where the metric value is with respect to a threshold range of the metric value.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates a diagram of a system for generating radiation therapy treatment plan alternatives for radiation therapy, according to an embodiment.
**FIG. 2** illustrates a flow diagram of a process for generating and evaluating radiation therapy treatment plans, according to an embodiment.
**FIG. 3A** illustrates a dose to volume diagram for an organ at risk, according to an embodiment.
**FIG. 3B** illustrates a cost function term generated for an example cost function in accordance with a dynamic heuristic parameter, according to an embodiment.
**FIG. 3C** illustrates the dynamic heuristic parameter of **FIG. 3B****.**
**FIG. 4** illustrates a dynamic heuristic parameter, according to an embodiment.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are configured to be considered within the scope of the subject matter disclosed herein. Other embodiments can be used and/or other changes can be made without departing from the scope of the present disclosure. The illustrative embodiments described in the detailed description are not meant to be limiting of the subject matter presented.

The systems and methods described, as well as the techniques they implement, improve conventional RT treatment planning techniques. More specifically, the systems and methods described enable clinicians to configure treatment planners based on an initial cost function that is based on both a kernel function usable to determine metrics associated with discrete doses of energy delivered to one or more portions of a patient and a modification function that cause the treatment planner update the metric values output by the kernel function and more quickly converge on a candidate treatment plan that is optimized for a set of metrics as compared to conventional treatment planners. And by configuring treatment planners to evaluate candidate treatment plans in accordance with a modification function as described herein, treatment planners can be configured to infer that certain aspects of treatment plans (alone or in combination) have a greater effect on the overall cost assigned to such treatment plans and adjust degrees of freedom accordingly when converging on an optimal candidate treatment plan. This can result in conserved computing resources as treatment planners no longer need to iterate through significant portions of (or the entirety of) a search space to identify an optimal treatment plan from among a set of possible candidate treatment plans. And by virtue of converging more quickly on optimal treatment plans, successive treatment plans can be more quickly evaluated by a given system, thereby reducing the downtime of a given LINAC while likewise reducing the time to treatment for each patient treated at a given facility.

Additionally, the presently-disclosed techniques address problems in the field when configuring treatment planners to calculate cost function gradients. Because treatment planner optimization often involves gradient calculation for individual metrics, treatment planners can encounter difficulty as values corresponding to metrics become infinitely smaller in a given parameter space (representing a distribution of possible degrees of freedom corresponding to candidate treatment plans). As an example, two example metrics for which a kernel function can be configured to generate metric values, dose at volume and volume at dose, are associated with gradients that can be difficult to interpret since the gradients are essentially a two-dimensional delta distribution located in a certain iso-dose surface. Issues arise when, for example, the equation representing dose at volume is discretized. Since the dose field and organ at risk volume definition is numerically presented in discretized form where the volume affecting a given metric is split into a set of voxels, it is more convenient to replace continuous versions of a volume-at-dose function (described below) with a discretized version: *VaD*(*D_{ref}*) = Σ_{*i*∈}*_{OAR} rᵢθ*(*Dᵢ - D_{ref}*), where *Dᵢ* is representing the dose level associated with a given voxel *i*, and *rᵢ* represent the volume of voxel *i* (or the part of volume in voxel *i* that corresponds to an organ at risk). The gradient of the step-function is non-zero only if its argument is zero, meaning that in practice the discretized equation for DaV will result in gradients that vanish. This can be an issue for treatment planners that rely on optimization algorithms such as gradient descent.

Because vanishing gradients can cause the gradient associated with a parameter space to indicate a direction of steepest ascent that becomes very small or even zero in certain regions, it can become increasingly difficult for treatment planners to determine a direction of steepest descent. As a result, when evaluating candidate treatment plans, the treatment planner can iterate across relatively small steps within the parameter space (resulting in inefficient traversal of the parameter space) or select a sub-optimal candidate treatment plan associated with (e.g., when caught within) a local minima. This can prevent the treatment planner from converging on an optimal candidate treatment plan and result in selection of a candidate treatment plan that involves the delivery of less energy to a planning target volume and/or more energy to non-targeted volumes (e.g., organs at risk). While certain approaches have been developed to replace these vanishing gradients with smoother gradients, these approaches generally involve identifying a vanishing gradient and replacing the metric value with a proxy metric value.

In some embodiments, treatment planners can be configured to replace the original value of a given metric when confronted with a vanishing gradient with a different value for the metric to improve performance of the treatment planner when addressing vanishing gradients. In this example, a treatment planner can be configured to identify this infinitely decreasing parameter and replace values beyond a threshold value with a default value that is constant. This default value can be determined by clinicians in various ways, balancing numerical performance (e.g., by using higher default values) and accurately presenting the original metric (e.g., by using lower default values). In an example, when calculating a volume-at-dose metric, the discretized version of the volume-at-dose function can be defined as follows: *VaD^{ε}*(*D_{ref}*) ≈ Σ_{*i*∈}*_{OAR} rᵢK^{ε}*(*Dᵢ - D_{ref}*) where the kernel function K^{ε}(*D*) is defined so that if *D* < *ε* or D > *ε*, the metric output by the discretized version of the volume-at-dose function corresponds to the step function, but in the vicinity of zero the metric is continuous and smooth. In essence, the original metric is replaced by an augmented metric that attempts to approximate the original metric while allowing the calculation of gradients more accurately. There are multiple choices for this function (for example arctan, sigmoid, trapezoid, or error-function) which each include introduction of a control parameter *ε* representing the width in dose space of the transition region *ε* → 0, *K^{ε}*(*D*) → *θ*(*D*) ∀ D). While the choice of *ε* > 0 solves issues related vanishing gradients, additional problems can arise. For example, values that are too small will lead to partially vanishing gradients where non-zero contribution is obtained only sporadically (typically leading to a situation where optimization algorithm does not find the local minimum since the gradient direction is not correct); and values that are too large cause *VaD^{ε}*(*D_{ref}*) to differ from the actual metric value *VaD*(*D_{ref}*) at an increasing rate, leading the optimizer implemented by the treatment planner to solve a problem that is different form the original problem and possibly lead to selection of a sub-optimal candidate treatment plan.

The present disclosure addresses these issues. For example, by virtue of the implementation of the techniques described herein, a cost determined for a given treatment plan can be modified in accordance with a dynamic value instead of a constant (as represented by the default values described above) such that higher or lower values can be introduced as a function of the value of the metric. This can, in turn, enable the treatment planner to more quickly (e.g., in fewer iterations) determine that candidate treatment plans associated with a given region within a parameter space are not optimal and forgo continued evaluation of candidate treatment plans associated with that region of the parameter space. This again reduces the computing resources needed to enable the treatment planner to converge on a candidate treatment plan. These techniques can also prevent treatment planners from focusing only on candidate treatment plans associated with a local minima in the region of the parameter space, allowing for the identification of candidate treatment plans outside of the local minima that can be better for the patient (e.g., lead to higher delivery of energy to planning target volumes and/or minimizing delivery of energy to organs at risk.

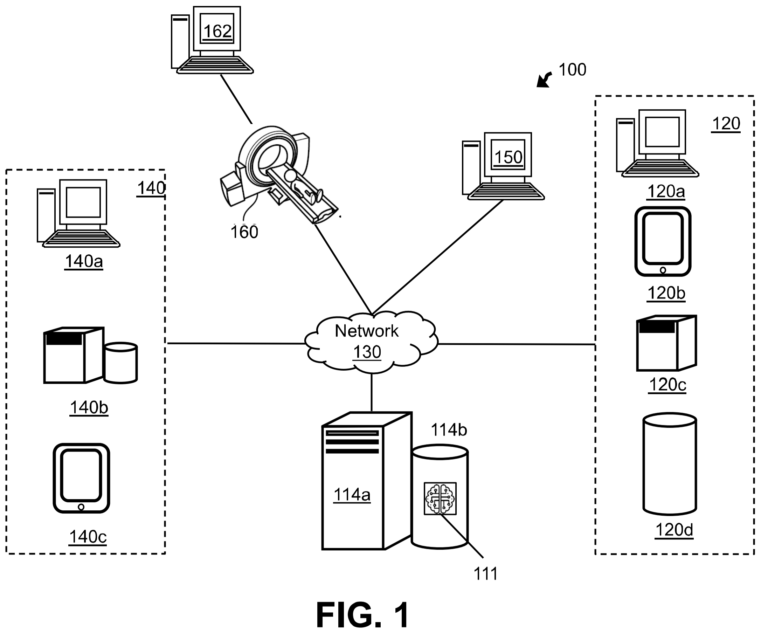

**FIG. 1** illustrates components of a system **100** for generating radiation therapy treatment plan alternatives for radiation therapy, according to an embodiment. The system **100** can include an analytics server **114a,** system database **114b,** a treatment planning system **111,** electronic data sources **120a-d** (each referred to individually as an electronic data source **120** and collectively electronic data sources **120,** unless stated otherwise), end-user devices **140a-c** (each referred to individually as an end user device **140** and collectively as end-user devices **140,** unless stated otherwise), an administrator computing device **150,** a medical device **160,** and medical device computer(s) **162.** Various components depicted in **FIG. 1** can belong to a radiotherapy clinic at which patients can receive radiotherapy treatment, in some cases via one or more radiotherapy machines located within the clinic (e.g., medical device **160**). The system **100** is not confined to the components described herein and can include additional or other components, not shown for brevity, which are configured to be considered within the scope of the embodiments described herein.

The above-mentioned components can be connected to each other through a network **130.** Examples of the network **130** can include, but are not limited to, private or public local-area-networks (LAN), wireless LAN (WLAN) networks, metropolitan area networks (MAN), wide-area networks (WAN), and the Internet. The network **130** can include wired and/or wireless communications according to one or more standards and/or via one or more transport mediums. The communication over the network **130** can be performed in accordance with various communication protocols such as Transmission Control Protocol and Internet Protocol (TCP/IP), User Datagram Protocol (UDP), and IEEE communication protocols. In one example, the network **130** can include wireless communications according to Bluetooth specification sets or another standard or proprietary wireless communication protocol. In another example, the network **130** can also include communications over a cellular network, including, e.g., a GSM (Global System for Mobile Communications), CDMA (Code Division Multiple Access), and EDGE (Enhanced Data for Global Evolution) network.

The analytics server **114a** can be any computing device comprising a processor and non-transitory machine-readable storage capable of executing the various tasks and processes described herein. The analytics server **114a** can employ various processors such as central processing units (CPU) and graphics processing unit (GPU), among others. Non-limiting examples of such computing devices can include workstation computers, laptop computers, server computers, and the like. While the system 100 includes a single analytics server **114a,** the analytics server **114a** can include any number of computing devices operating in a distributed computing environment, such as a cloud environment.

The analytics server **114a** can generate and display an electronic platform configured to use a treatment planning system **111** for receiving patient information, inputs from users (e.g., clinicians) such as utility functions and updated utility functions described herein, and outputting the results of execution of the treatment planning system **111.** The electronic platform can include graphical user interfaces (GUI) displayed by display devices of one or more electronic data sources **120,** the end-user devices **140,** the medical device **160,** and/or the administrator computing device **150.** An example of the electronic platform generated and hosted by the analytics server **114a** can be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the treatment planning system **111.** For instance, the analytics server **114a** can execute the treatment planning system **111** (e.g., a system such as a treatment planner that is configured and/or trained to generate fluence maps, leaf sequences, etc., as described herein for a patient being treated via the medical device **160**). The analytics server **114a** can then display the results for a clinician and/or directly revise one or more operational attributes of the medical device **160.**

The electronic data sources **120** can be any computing device comprising a processor and non-transitory machine-readable storage capable of executing the various tasks and processes described herein. For example, the electronic data sources **120** can represent various computing devices that contain, retrieve, and/or access data associated with a medical device **160,** such as data associated with operational information of currently or previously performed radiotherapy treatments (e.g., electronic log files or electronic configuration files), data associated with current and/or previously monitored patients (e.g., computed tomography (CT) scans, magnetic resonance imaging (MRI) scans, tumor locations, deformation information, and/or the like) or participants in a study, and/or the like. For instance, the analytics server **114a** can use the clinic computer **120a,** medical professional device **120b,** server **120c** (associated with a clinician and/or a clinic), and database **120d** (associated with the clinician and/or the clinic) to retrieve/receive data associated with the medical device **160.** The analytics server **114a** can retrieve the data from the end-user devices **120,** generate a dataset, and use the dataset to configure the treatment planning system **111** (e.g., models implemented by the treatment planning system **111** and/or the like). The analytics server **114a** can execute various algorithms to translate raw data received/retrieved from the electronic data sources **120** into machine-readable objects that can be stored and processed by other analytical processes as described herein.

End-user devices **140** can be any computing device comprising a processor and a non-transitory machine-readable storage medium capable of performing the various tasks and processes described herein. Non-limiting examples of an end-user device **140** can be a workstation computer, laptop computer, tablet computer, or server computer. In operation, various users such as clinicians as described herein can use end-user devices **140** to access the GUI operationally managed by the analytics server **114a** or otherwise the results of the execution of the treatment planning system **111.** Specifically, the end-user devices **140** can include clinic computer **140a,** clinic server **140b,** and a medical professional device **140c.** Even though referred to herein as "end-user" devices, these devices can not always be operated by end-users. For instance, the clinic server **140b** can not be directly used by an end user. However, the results stored on the clinic server **140b** can be used to populate various GUIs accessed by an end user via the medical professional device **140c.** In some embodiments, the end-user device **140** can be associated with one or more clinicians that are associated with the generation of one or more treatment plans (e.g., involved in preparing the one or more treatment plans) for patients.

The administrator computing device **150** can represent a computing device operated by a system administrator. The administrator computing device **150** can be configured to display radiotherapy treatment attributes generated by the analytics server **114a** (e.g., various analytic metrics determined during training of one or more machine learning models and/or systems); monitor various treatment planning systems **111** utilized by the analytics server **114a,** electronic data sources **120,** and/or end-user devices **140;** review feedback; and/or facilitate training or retraining (calibration) of the treatment planning system **111** that are maintained by the analytics server **114a.**

In some embodiments, the medical device **160** can be a diagnostic imaging device or a treatment delivery device. For example, the medical device **160** can include one or more computed tomography (CT) scanners, linear accelerators (LINACs) having a multi-leaf collimator (MLC) that consists of multiple small lead leaves that can be individually moved to shape the radiation beam and deliver the dose to the tumor while minimizing the dose to surrounding healthy tissues, or other similar devices configured to transmit energy toward targeted tissue (referred to as planning target volumes) associated with a patient and, in some cases, measure the energy transferred toward the targeted tissue. The medical device **160** can also include one or more sensors configured to monitor the patient being treated. That is, the medical device **160** and/or the analytics server **114a** can be communicating with various sensors that can monitor a patient's external biological signals. Non-limiting examples of the sensors can include 3D surfacing mechanisms and optical (or other) sensors configured to monitor the patient's movements (e.g., how the patient is moving and/or breathing). In some embodiments, the medical device **160** can receive data associated with a treatment plan from the medical device computer(s) **162** that cause the medical device **160** to operate in accordance with the treatment plan.

The treatment planning system **111** can be stored in the system database **114b.** The treatment planning system **111** can be trained using data received/retrieved from the electronic data sources **120** and can be executed using data received from the end-user devices, the medical device **160,** and/or the sensor **163.** In some embodiments, the treatment planning system **111** can reside within a data repository local or specific to a clinic. In various embodiments, the treatment planning system **111** can use one or more deep learning engines to develop a treatment plan for a patient using radiation therapy. For instance, the analytics server **114a** can transmit patient attributes from the sensor **163** and execute the treatment planning system **111** accordingly. The analytics server **114a** can then display the results on one or more end-user devices **140.** In some embodiments, the analytics server **114a** can change one or more configurations of the medical device **160** based on the results predicted by the treatment planning system **111.**

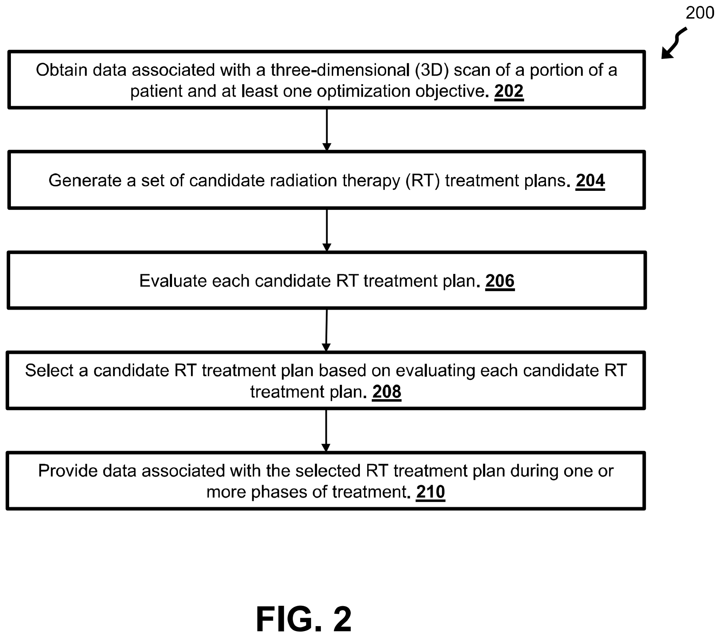

Referring to **FIG. 2****,** illustrated is a flow diagram of a process **200** for generating and evaluating radiation therapy treatment plans. The process **200** includes operations **202-210.** However, other embodiments can include additional or alternative operations or can omit one or more operations altogether. The process **200** is described as being executed by an analytics server, which can be the same as, or similar to, the analytics server **114a** described in **FIG. 1****.** However, one or more steps of the process **200** can be executed by any number of computing devices operating in the distributed computing system described in **FIG. 1****.** For instance, one or more computing devices can locally perform part or all of the operations described in **FIG. 2****.**

At operation **202,** the analytics server can obtain data associated with a three-dimensional (3D) scan of a portion of a patient and at least one optimization objective. For example, the analytics server can obtain data generated by a medical imaging device (e.g., a computed tomography (CT) scanner and/or the like) representing at least a portion of a patient. In this example, the 3D scan can represent a planning target volume (PTV) such as a tumor or other area where the delivery of energy (e.g., using a LINAC as described herein) is desired to treat the patient. The 3D scan can also represent one or more anatomical features that are different from the PTV. For example, the 3D scan can represent one or more organs in proximity to the PTV that can receive energy during delivery of energy by a LINAC to the PTV but are not associated with the PTV. In this example, the one or more organs are referred to as organs at risk (OARs). It will be understood that the treatment objective of a given RT treatment plan can be to maximize the amount of energy delivered to a PTV while minimizing the amount of energy delivered to the OARs.

In some embodiments, the at least one treatment objective can represent one or more desired aspects of delivery of RT treatment of the patient. For example, the at least one treatment objective can include one or more of target coverage (e.g., a volume of the PTV to receive a prescribed amount of radiation as compared to the entire PTV), a tumor eradication rate (e.g., a probability that all of the cancer cells in the PTV will be eliminated), satisfying an amount of energy delivered to a PTV (e.g., as measured by an amount of energy delivered to the entire PTV), satisfying a maximum amount of energy delivered to an OAR (e.g., as measured by an amount of energy delivered to the OAR), conformance of energy delivery to the PTV (e.g., an amount of the PTV receiving energy delivered at a given rate as compared to the total volume of the PTV), establishing homogeneity of energy delivery to the PTV (e.g., based on determination of a homogeneity index), and/or the like. The at least one treatment objective can be determined based on input by a clinician specifying the at least one treatment objective. For example, a treatment objective associated with delivery of energy to OARs can be specified by a clinician based on the clinician providing input (e.g., at an end user device that is the same as, or similar to, the end user devices **140** of **FIG. 1**) indicating that the relative volume of the OAR where more than 50 Gy energy was delivered should not exceed 10% and that candidate treatment plans should not be accepted when the aforementioned relative volume exceeds 20%.

At operation **204,** the analytics server can generate a set of candidate RT treatment plans based on the 3D scan of the portion of the patient. For example, the analytics server can determine one or more PTVs and one or more OARs based on the 3D scan of at least a portion of the patient. In this example, the analytics server can determine one or more boundaries representing extents of the PTVs and OARs in the 3D scan based on input by a clinician indicating the boundaries and/or based on the analytics server executing a segmentation system. The segmentation system can include software and/or hardware configured to receive the 3D scan and output an indication of the extents of the PTVs and OARs represented by the 3D scan. The analytics server can then define an initial beam configuration specifying, for example, a number of beams, angles of incidence for the beams, and initial beam shapes as an initial candidate treatment plan and make adjustments to the initial beam configuration to determine a candidate treatment plan. In some embodiments, the analytics server can evaluate the candidate treatment plan as described herein and iteratively adjust the successively-generated candidate treatment plans to converge on a candidate treatment plan having a lowest cost.

At operation **206,** the analytics server can evaluate each candidate RT treatment plan. In some embodiments, the analytics server can evaluate each candidate RT treatment plan based on the analytics server determining one or more metrics (measured using one or more metric values) that correspond to one or more treatment objectives for each candidate treatment plan. For example, the analytics server can simulate operation of a LINAC delivering energy to a PTV in accordance with each candidate treatment plan and determine metrics representing, for example, expected amounts of energy delivered to portions of the PTV and OARs (e.g., to voxels corresponding to the PTV and OARs). The analytics server can then determine the metrics based on the simulated energy delivered to the PTV and to the OARs and select a candidate treatment plan as an optimal treatment plan. Metrics can include, for example, any combination of one or more of: target underdosing (e.g., a value indicating a degree to which portions of a PTV receive a dose below a prescribed level), OAR overdosing (e.g., a degree to which portions of an OAR receive a dose above a prescribed level), plan complexity (e.g., a relative number of beam positions and configurations when compared to other candidate RT treatment plans), and/or the like. In some embodiments, two example metrics can include a volume-at-dose (VaD) and dose-at-volume (DaV). In continuum level these metrics can be defined as follows: 1 (Volume-at-dose): *VaD^{c}*(*D_{ref}*) = *∫*_{*x*∈}*_{OAR} dx θ*(*D*(*x*) *- D_{ref}*); 2 (Dose-at-volume): *V_{ref}* = *∫*_{*x*∈}*_{OAR} dx θ*(*D*(*x*) *- DaV^{c}*(*V_{ref}*)); where *D*(*x*) is dose at location x and *θ*(*D*) is a step function being either 1 (when D>0) or 0. It will be understood that DaV is defined implicitly in the above equation, but are similar in form. The present disclosure focuses on the VaD for purposes of simplicity, but all aspects demonstrated with VaD could be repeated also for DaV. Additionally, the Dₘₐₓ metric is a special case of DaV with zero or very small volume. As described herein, an optimal treatment plan can include a candidate treatment plan that has a cost that satisfies a threshold value (e.g., a candidate RT treatment plan having a cost that is lower than one or more other candidate RT treatment plans).

In some embodiments, the analytics server can determine the cost for each single point dose (e.g., discrete doses at which energy is delivered to at least a portion of the patient) of the candidate RT treatment plans based on a kernel function associated with a metric evaluation function that is included as a part of the cost function. For example, the analytics server can determine the cost for each of the candidate RT treatment plans based on a cost function that includes a metric evaluation function represented using one or more kernel functions and a modification function (represented herein as a set of dynamic heuristic parameters represented as one or more epsilon (*ε*) values). In this example, metric evaluation function can include a one or more piecewise linear functions (referred to as kernel functions) that are determined based on the metrics for the candidate RT treatment plans, and the modification function can include one or more values configured to modify the corresponding outputs of the one or more metrics related to one or more piecewise linear functions based on the value associated with metrics. As will be understood, when a metric value corresponding to a given metric satisfies a threshold range of values for that metric when evaluated in accordance with the metric evaluation function described herein, the modification function can update the output of the metric evaluation function (e.g., by updating the values associated with evaluation of one or more respective kernel functions) such that subsequent evaluation of costs to increase or decrease as the degree to which the values for the given metric exceeds the threshold range of values for that metric. This, in turn, can cause the cost of a given treatment plan to be modified at one or more single point doses to allow evaluation of the treatment plans by the analytics server to converge on a candidate RT treatment plan quicker than would otherwise be possible or slower where more it may be desirable to perform more accurate metric calculations.

As described above, the cost function can be represented as a set of piecewise linear functions corresponding to metrics used to evaluate each of the candidate RT treatment plans. To evaluate each candidate RT treatment plan, the analytics server can determine a value for each of the piecewise linear functions corresponding to each candidate treatment plan and add the values in accordance with the metric evaluation function network to determine an overall cost for each candidate RT treatment plan. The value output by the metric evaluation function can represent a degree to which a given candidate RT treatment plan satisfies the desired goals (target coverage, OAR sparing, and/or the like) when treating a patient, where higher costs are associated with lesser degrees of satisfaction (e.g., lower target coverage, higher OAR sparing, and/or the like).

In some embodiments, the modification function can be based on the at least one optimization objective. For example, the modification function can be based on the at least one optimization objective indicated by input provided by a clinician to the analytics server. In another example, the modification function can be based on the at least one optimization objective, where the at least one optimization objective is set as a predetermined optimization objective by the analytics server. Examples of optimization objectives can include delivering an amount of energy to a threshold portion of a PTV, not exceeding an amount of energy delivered to one or more OARs, achieving conformance of the PTV when delivering the energy, achieving a uniform distribution of energy throughout the PTV and/or the like.

In some embodiments, the analytics server can evaluate each candidate RT treatment plan based on a cost function such that the values output by the kernel function(s) of the metric evaluation function are updated based on changes in values output by the cost function for individual metrics or for a set of metrics where a given metric satisfies a threshold range of values. For example, a kernel function can include a modification function having a first factor and a second factor. The first factor of the modification function can correspond to a given metric satisfying a first threshold range and the second factor of the modification function can correspond to metrics satisfying a second threshold range. In one illustrative example, where the metric represents a percentage of a rectum of a patient receiving 50Gy as a result of a given candidate RT treatment plan, the first threshold range can include between 10 and 20% of the rectum of the patient receiving 50Gy and the second threshold range can include more than 20% of the rectum of the patient receiving 50Gy. In this example, where an optimization objective is to minimize energy delivered to the rectum of the patient (e.g., where the rectum is an OAR), the analytics server can determine the first factor to be a value that is lower than the second factor. The analytics server can then evaluate each of the candidate RT treatment plans such that the cost for each candidate RT treatment plan is updated (e.g., multiplied) based on the first factor where 50Gy is delivered to between 10 and 20% of the rectum of the patient, and updated (e.g., multiplied) by the second factor where 50Gy is delivered to more than 20% of the rectum of the patient. In some embodiments, the modification function can further include a third factor corresponding to the given metric satisfying a third threshold range. In the above illustrative example, the third threshold range can include less than 10% of the rectum of the patient receiving 50Gy, and the third factor can be a value that is greater than the first factor and/or the second factor. In this way, as the energy delivered to the rectum approaches zero, the analytics server can update the value so as to avoid a local minima.

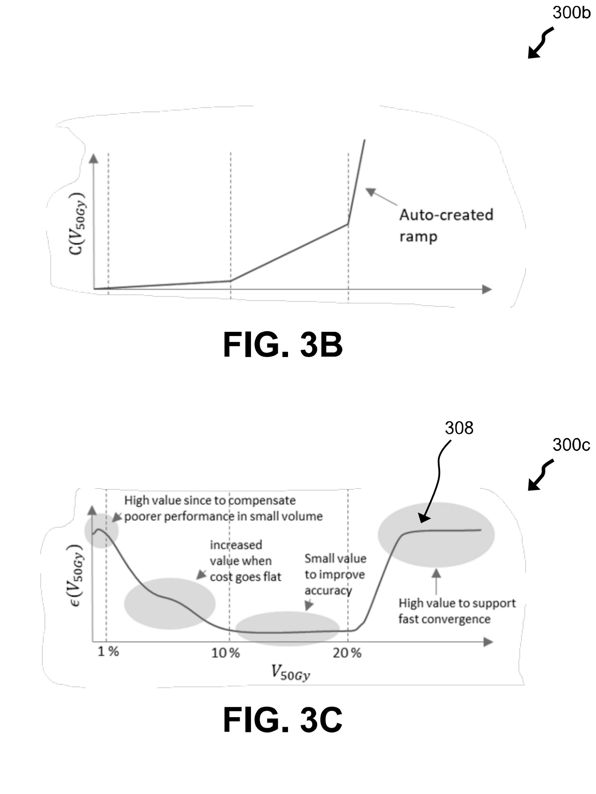

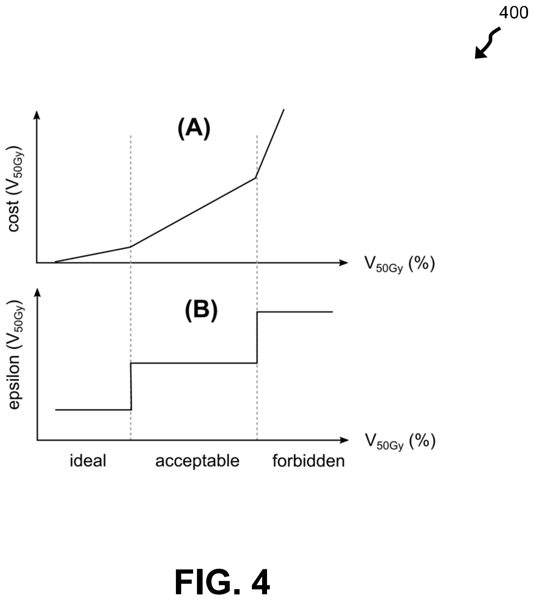

In some embodiments, the first factor, second factor, and/or third factor can include a set of values associated with a function (also referred to as a functional form). For example, the first factor, second factor, and/or third factor can include a set of values that are based on the metrics. In the above illustrative example, the first factor can include a range of values corresponding to the amount of energy delivered that remain relatively constant and begin to increase as the amount of energy approaches 10% or 20% (the bounds of the first threshold range), the second factor can include a range of values that increase as the amount of energy increases beyond 20%, and the third factor can include a range of values that increase as the amount of energy decreases. The ranges of values corresponding to the first factor, the second factor, and third factor are illustrated as sets of example values with respect to the value for (*ε*) shown in **FIG. 3B****.** In another illustrative example, the first factor, second factor, and/or third factor can include a value for (*ε*) that remains constant, as shown in **FIG. 4****.** It will be understood that, in some embodiments, the first factor, second factor, and/or third factor can include corresponding combinations of variable and constant values for each of the first factor, the second factor, and/or the third factor.

At operation **208,** the analytics server can select a candidate RT treatment plan based on evaluating each candidate RT treatment plan. For example, the analytics server can select a candidate RT treatment plan based on the cost for each of the candidate RT treatment plans determined based on the kernel function. In this example, when selecting the candidate RT treatment plan, the analytics server can compare the cost for each of the candidate RT treatment plans to the cost of one or more other candidate RT treatment plans to determine (e.g., identify) a candidate treatment plan having a cost that satisfies a threshold value. The threshold value can include a value that is the lowest value associated with a candidate RT treatment plan when compared to one or more other candidate RT treatment plans.

At operation **210** the analytics server can provide data associated with the selected candidate RT treatment plan during one or more phases of treatment of the patient. For example, the analytics server can provide the data associated with the selected candidate RT treatment plan to a medical device computer (e.g., a medical device computer that is the same as, or similar to, the medical device computer(s) **162** of **FIG. 1**) that is configured to control operation of a medical device (e.g., a medical device that is the same as, or similar to, the medical device **160** of **FIG. 1**). The data associated with the selected candidate RT treatment plan can be configured to cause a medical device to perform one or more operations. For example, where the medical device includes a LINAC, the data can be configured to cause a gantry of the LINAC to move such that a treatment head is positioned to deliver energy in accordance with one or more control points at one or more power levels.

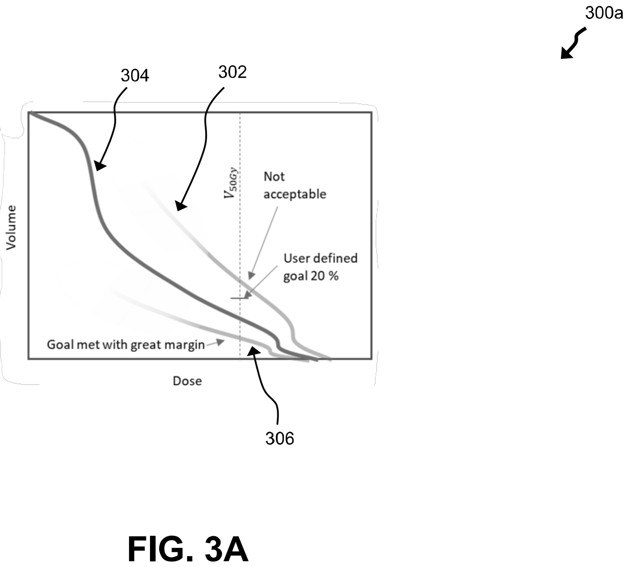

Referring now to **FIGS. 3A-3C** and **FIG. 4****,** an example analysis of candidate RT treatment plans is provided. More specifically, **FIG. 3A** is an example OAR dose-volume-histogram **300a** for an anatomical feature of a patient (e.g., a rectum) when treated in accordance with a first candidate RT treatment plan **302,** a second candidate RT treatment plan **304,** and a third candidate RT treatment plan **306,** **FIG. 3B** is a representation of a cost function term **300b** of the metric represented by **FIG. 3A****,** and **FIG. 3C** is a functional form (set of *ε* parameters) for a modification function **300c.** **FIG. 4** illustrates an alternative functional form for a modification function **400** that can be used in place of the modification function **300c of** **FIG. 3C** where the set of parameters represented by the functional form are constant within predetermined ranges.

With continued reference to **FIGS. 3A-3C** and **FIG. 4****,** in some embodiments, a kernel function can be represented as *K*(*D, ε*(*VaD*(*D_{ref}*))), where *VaD* refers to a metric calculated using a cost function **300b** (e.g., by the analytics server) to determine a value corresponding to a metric and represents the values associated with a modification function **300c.** In an example, an analytics server (e.g., that is the same as, or similar to, the analytics server **114a** of **FIG. 1**) can determine a value for a metric of the kernel function when evaluating candidate RT treatment plans as described herein, where the value for the metric is based on (e.g., represents) delivery of 50Gy to a rectum of a patient (*V*_{50*Gy*}) during treatment. In this example, the rectum can represent an example of an OAR. The analytics server can also receive input from a clinician indicating that the value for the metric needs to be less than a threshold amount (e.g., 20%) to satisfy an optimization objective. In this example, the analytics server can determine (e.g., construct) the cost function as illustrated in **FIG. 3B** to take into account this indication that the metric needs to be less than the threshold amount to satisfy the optimization objective and determine a modification function for that corresponds to this optimization objective.

As illustrated in **FIG. 3C****,** the analytics server can determine the modification function such that the function **308** is represented using a continuous set of *ε* values. Accordingly, when evaluating candidate RT treatment plans, the analytics server can determine the cost for each candidate treatment plan based on the cost function depending on the selected kernel function and, for candidate RT treatment plans where the value for the metric does not satisfy the optimization objective, the cost increases at a predetermined rate. This accelerated increase in cost that is, at least in part, attributable to the modification function **308** when candidate treatment plans do not satisfy the optimization objective (e.g., exceed 50Gy) allows the analytics server to more quickly converge on an optimal candidate RT treatment plan (e.g., candidate RT treatment plan **306**) without setting a threshold cost that would prevent further evaluation of the other metrics (and ultimate cost of the candidate treatment plan) is to indicate how to improve the plan so that the acceptable region is reached.

With continued reference to **FIG. 3C****,** the epsilon of the kernel function can be represented as a functional form presented such that, for the region *V*_{50*Gy*} > 20%, the analytics server sets the epsilon value to a series of increasing values to enable the treatment planner implemented by the analytics server to iterate at an increased rate (e.g., an exponential rate, a logarithmic rate, and/or the like) away from the unacceptable region to the acceptable region. In the interval [10%,20%], the analytics server sets the epsilon value such that the values associated with the interval [10%,20%] more closely approximate the true value (e.g., that *V*_{50*Gy*} (*ε*, *D*) ≈ *V*_{50*Gy*}(*D*)). Once *V*_{50*Gy*} < 10% the analytics server again sets the epsilon value at higher values relative to the values associated with [10%,20%] to handle potential vanishing or exploding gradients as the cost is reduced, especially once *V*_{50*Gy*} < 1% where the values associated with the metric can have encounter stability issues (e.g., as the gradient vanishes approaching 0). Alternatively, as illustrated by **FIG. 4****,** the analytics server can set the epsilon value to a series of constant values associated with one or more thresholds for the metric. This is illustrated in **FIG. 4** as a step function, whereby epsilon values associated with a modification function increase in a stepwise fashion as the delivery of 50Gy to a portion of a patient increases. As such, as a treatment planner iterates through treatment plans within a given portion of a parameter space where the amount of energy delivered to a given anatomical structure is ideal (e.g., below a threshold amount), the modification function updates the output of the kernel function to a lesser degree (e.g., in accordance with a relatively lower epsilon value) as opposed to iterations by the treatment planner through treatment plans within a different portion of the parameter space where the amount of energy delivered to a given anatomical structure is acceptable (e.g., at or above the threshold amount) where the modification function updates the output of the kernel function to a greater degree (e.g., in accordance with a relatively higher epsilon value). Similarly, as the treatment planner iterates through treatment plans within a given portion of the parameter space where the amount of energy delivered to a given anatomical structure is not ideal or forbidden (e.g., above a separate, higher threshold amount), the modification function updates the output of the kernel function to an even greater degree (e.g., in accordance with an epsilon value that is higher than the epsilon values associated with the other portions of the parameter space).

The various illustrative logical blocks, modules, circuits, and algorithm steps described in connection with the embodiments disclosed herein can be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans can implement the described functionality in varying ways for each particular application, but such implementation decisions should not be interpreted as causing a departure from the scope of this disclosure or the claims.

Embodiments implemented in computer software can be implemented in software, firmware, middleware, microcode, hardware description languages, or any combination thereof. A code segment or machine-executable instructions can represent a procedure, a function, a subprogram, a program, a routine, a subroutine, a module, a software package, a class, or any combination of instructions, data structures, or program statements. A code segment can be coupled to another code segment or a hardware circuit by passing and/or receiving information, data, arguments, parameters, or memory contents. Information, arguments, parameters, data, etc., can be passed, forwarded, or transmitted via any suitable means, including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions can be stored as one or more instructions or code on a non-transitory computer-readable or processor-readable storage medium. The steps of a method or algorithm disclosed herein can be embodied in a processor-executable software module, which can reside on a computer-readable or processor-readable storage medium. A non-transitory computer-readable or processor-readable media includes both computer storage media and tangible storage media that facilitate the transfer of a computer program from one place to another. A non-transitory processor-readable storage media can be any available media that can be accessed by a computer. By way of example, and not limitation, such non-transitory processor-readable media can comprise RAM, ROM, EEPROM, CD-ROM or other optical disk storage, magnetic disk storage or other magnetic storage devices, or any other tangible storage medium that can be used to store desired program code in the form of instructions or data structures and that can be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm can reside as one or any combination or set of codes and/or instructions on a non-transitory processor-readable medium and/or computer-readable medium, which can be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein can be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A system comprising:
one or more processors configured to:
obtain data associated with a three-dimensional, 3D, scan of a portion of a patient and at least one optimization objective;
generate a set of radiation therapy, RT, treatment plans based on the 3D scan of the portion of the patient;
evaluate each RT treatment plan of the set of RT treatment plans based on a kernel function to generate RT treatment plan costs for each corresponding RT treatment plan, where evaluation of each RT treatment plan involves updating an output of the kernel function for each RT treatment plan based on a modification function that is based on the at least one optimization objective;
determine a selected RT treatment plan from among the set of RT treatment plans based on comparing the RT treatment plan costs of the selected RT treatment plan to the RT treatment plan costs of each of the RT treatment plans; and
providing data associated with the selected RT treatment plan to control operation of a linear accelerator, LINAC, during one or more phases of treatment of the patient.

2. The system of claim 1, wherein the one or more processors configured to evaluate each RT treatment plan of the set of RT treatment plans are configured to:
select the kernel function based on one or more aspects associated with a planning target volume represented by the 3D scan; and
evaluate each RT treatment plan based on the kernel function, where the modification function is configured to update metric values output by the kernel function that are outside of a threshold range of metric values.

3. The system of claim 2, wherein the one or more processors configured to update the metric values output by the kernel function outside of the threshold range of metric values are configured to:
update the metric values outside of the threshold range by a first factor such that consecutive metric values outside of the threshold range are updated at an exponential or logarithmic rate.

4. The system of claim 2 or claim 3, wherein the one or more processors configured to evaluate each RT treatment plan of the set of RT treatment plans are configured to:
evaluate each RT treatment plan based on the kernel function, where the modification function is configured to update a first set of values output by the kernel function that are outside of the threshold range of metric values by a first factor, and a second set of values output by the kernel function that are outside of the threshold range of metric values by a second factor.

5. The system of claim 4, wherein the one or more processors configured to evaluate each RT treatment plan of the set of RT treatment plans are configured to:
evaluate each RT treatment plan based on the kernel function, where the modification function is configured to update a third set of values that satisfy the threshold range of metric value by a third factor that is less than the first factor and the second factor.

6. The system of any preceding claim, wherein the one or more processors configured to evaluate each RT treatment plan of the set of RT treatment plans are configured to:
evaluate each RT treatment plan based on the kernel function, where the modification function is configured to update metric values output by the kernel function based on a functional form of a cost function;
and optionally; wherein the functional form of the cost function represents rates of change corresponding to dose values of energy delivered to a planning target volume or an organ at risk.

7. A method comprising:
obtaining, by at least one processor, data associated with a three-dimensional, 3D, scan of a portion of a patient and at least one optimization objective;
generating, by the at least one processor, a set of radiation therapy, RT, treatment plans based on the 3D scan of the portion of the patient;
evaluating, by the at least one processor, each RT treatment plan of the set of RT treatment plans based on a kernel function to generate RT treatment plan costs for each corresponding RT treatment plan, where evaluation of each RT treatment plan involves updating an output of the kernel function for each RT treatment plan based on a modification function that is based on the at least one optimization objective; and
determining, by the at least one processor, a selected RT treatment plan from among the set of RT treatment plans based on comparing the RT treatment plan costs of the selected RT treatment plan to the RT treatment plan costs of each of the RT treatment plans.

8. The method of claim 7, wherein evaluating each RT treatment plan of the set of RT treatment plans comprises:
selecting, by the at least one processor, the kernel function based on one or more aspects associated with a planning target volume represented by the 3D scan; and
evaluating, by the at least one processor, each RT treatment plan based on the kernel function, where the modification function is configured to update metric values output by the kernel function that are outside of a threshold range of metric values.

9. The method of claim 8, wherein updating the metric values output by the kernel function outside of the threshold range of metric values comprises:
updating, by the at least one processor, the metric values outside of the threshold range by a first factor such that consecutive metric values outside of the threshold range are updated at an exponential or logarithmic rate.

10. The method of claim 8 or claim 9, wherein evaluating each RT treatment plan of the set of RT treatment plans comprises:
evaluating, by the at least one processor, each RT treatment plan based on the kernel function, where the modification function is configured to update a first set of values output by the kernel function that are outside of the threshold range of metric values by a first factor, and a second set of values output by the kernel function that are outside of the threshold range of metric values by a second factor;
and optionally:
wherein evaluating each RT treatment plan of the set of RT treatment plans comprises:
evaluating, by the at least one processor, each RT treatment plan based on the kernel function, where the modification function is configured to update a third set of values that satisfy the threshold range of metric value by a third factor that is less than the first factor and the second factor.

11. The method of any of claim 7 to claim 10, wherein evaluating each RT treatment plan of the set of RT treatment plans comprises:
evaluating, by the at least one processor, each RT treatment plan based on the kernel function, where the modification function is configured to update metric values output by the kernel function based on a functional form associated with the modification function;
and optionally:
wherein the functional form associated with the modification function represents rates of change corresponding to dose values of energy delivered to a planning target volume or an organ at risk.

12. A non-transitory computer-readable medium storing instructions thereon that, when executed by at least one processor, causes the at least one processor to:
obtain data associated with a three-dimensional, 3D, scan of a portion of a patient and at least one optimization objective;
generate a set of radiation therapy, RT, treatment plans based on the 3D scan of the portion of the patient;
evaluate each RT treatment plan of the set of RT treatment plans based on a kernel function to generate RT treatment plan costs for each corresponding RT treatment plan, where evaluation of each RT treatment plan involves updating an output of the kernel function for each RT treatment plan based on a modification function that is based on the at least one optimization objective;
determine a selected RT treatment plan from among the set of RT treatment plans based on comparing the RT treatment plan costs of the selected RT treatment plan to the RT treatment plan costs of each of the RT treatment plans; and
provide data associated with the selected RT treatment plan to control operation of a linear accelerator, LINAC, during one or more phases of treatment of the patient.

13. The non-transitory computer-readable medium of claim 12, wherein the instructions that cause the one or more processors to evaluate each RT treatment plan of the set of RT treatment plans cause the one or more processors to:
select the kernel function based on one or more aspects associated with a planning target volume represented by the 3D scan; and
evaluate each RT treatment plan based on the kernel function, where the modification function is configured to update metric values output by the kernel function that are outside of a threshold range of metric values;
and optionally:
wherein the instructions that cause the one or more processors to update the metric values output by the kernel function outside of the threshold range of metric values cause the one or more processors to:
update the metric values outside of the threshold range by a first factor such that consecutive metric values outside of the threshold range are updated at an exponential or logarithmic rate.

14. The non-transitory computer-readable medium of claim 13, wherein the instructions that cause the one or more processors to evaluate each RT treatment plan of the set of RT treatment plans cause the one or more processors to:
evaluate each RT treatment plan based on the kernel function, where the modification function is configured to update a first set of values output by the kernel function that are outside of the threshold range of metric values by a first factor, and a second set of values output by the kernel function that are outside of the threshold range of metric values by a second factor;
and optionally:
wherein the instructions that cause the one or more processors to evaluate each RT treatment plan of the set of RT treatment plans cause the one or more processors to:
evaluate each RT treatment plan based on the kernel function, where the modification function is configured to update a third set of values that satisfy the threshold range of metric value by a third factor that is less than the first factor and the second factor.

15. The non-transitory computer-readable medium of any of claim 12 to claim 14, wherein the instructions that cause the one or more processors to evaluate each RT treatment plan of the set of RT treatment plans cause the one or more processors to:
evaluate each RT treatment plan based on the kernel function, where the modification function is configured to update metric values output by the kernel function based on a functional form associated with the modification function.
